Europäisches Patentamt

(19) **European Patent Office**

Office européen des brevets

(11) Publication number: **0 036 258**
**A2**

(12) **EUROPEAN PATENT APPLICATION**

(21) Application number: **81300857.0**

(22) Date of filing: **02.03.81**

(51) Int. Cl.³: **C 12 P 21/02**
**C 12 N 15/00, C 07 C 103/52**
**C 12 N 1/20**
**//C12R1/19, A23L1/236**

(30) Priority: **14.03.80 US 130462**

(43) Date of publication of application:
**23.09.81 Bulletin 81/38**

(84) Designated Contracting States:
**AT BE CH DE FR GB IT LI LU NL SE**

(71) Applicant: **CETUS CORPORATION**
**600 Bancroft Way**
**Berkeley, California(US)**

(72) Inventor: **Bahl, Chander Prakash**
**908, Norwell Street**
**El Cerrito(US)**

(72) Inventor: **Rose, Janine Estelle**
**4435 Fieldcrest Drive**
**Richmond California(US)**

(72) Inventor: **White, Thomas James**
**2632 Etna Street, Apt. A**
**Berkeley California(US)**

(74) Representative: **Allard, Susan Joyce et al,**
**BOULT, WADE & TENNANT 27 Furnival street**
**London EC4A 1PQ(GB)**

(54) **Process for producing aspartame.**

(57) The artificial sweetener aspartame, a dipeptide with the formula Asp-Phe-me, is produced using a cloned microorganism. A DNA which codes for a large stable peptide comprised of the repeating amino acid sequence (Asp-Phe)n is inserted into a cloning vehicle which in turn is introduced into a suitable host microorganism. The host microorganism is cultured and the large peptide containing the repeating Asp-Phe sequence is harvested therefrom. The free carboxyl group of the large peptide is benzylated and then hydrolysed to benzyl Asp-Phe dipeptides. This dipeptide is methylated and then debenzylated to form aspartame.

EP 0 036 258 A2

- 1 -

PROCESS FOR PRODUCING ASPARTAME

This invention relates to the production of artificial sweeteners and more particularly to the production of sweet tasting peptides through the use of genetically manipulated microorganisms.

The most widely used and cheapest sweetener presently available is sucrose, generally derived from cane or beet. However, some people such as diabetics must severely limit or abstain from the consumption of sucrose. Moreover, medically deleterious effects of sucrose are being described increasingly in medical journals as well as in the popular press. Not least of the harmful effects of sucrose is its contribution to the problem of obesity.

Various so-called artificial sweeteners have been developed as a substitute for sucrose. At one time such sweeteners as saccharine and the cyclamates were widely distributed and held promise as a means of limiting consumption of sucrose. Recent research, however, has led to the suggestion that saccharine and cyclamates may be carcinogenic and, consequently, the government is questioning their use for many purposes. The prohibitions on use of such sweeteners have forced many food processors to return to the use of sucrose. This has created significant inconvenience for those persons such as diabetics, who cannot tolerate sucrose, and for those persons trying to control their weight.

A dipeptide, having the structure Asp-Phe-me, is described in Patent No. 3,492,131 issued to J. H. Schlatter. This dipeptide, aspartame, has been found to be from 100 to 200 times sweeter than sucrose. Aspartame is not only sweeter than sucrose, but is preferable as a food to sucrose. While sucrose can provide the body with little more than energy, aspartame is composed of amino acids, the building blocks of body

proteins, and like other proteins is broken down by the digestive enzymes in the stomach to its constituent amino acids thus providing nutritive value. This fact also makes it unlikely that aspartame will be found to have carcinogenic properties, such as have been found in saccharine and cyclamates which are not similarly digested.

For these reasons, aspartame holds significant promise in replacing sugar as a sweetener. However, because sucrose is a relatively inexpensive substance, aspartame, if it is to gain widespread commercial acceptance, must be produced for a price which is reasonably competitive with sucrose.

One way of producing aspartame is by using known peptide synthesis techniques. However, the synthesis of specific peptide chains is generally a tedious and expensive process. While amino acids such as aspartic acid and phenylalanine are readily available and while the formation of peptide bonds is easily achieved, correct synthesis of peptide sequences involves intensive protecting and deprotecting of alpha amino, alpha carboxyl and side chain groups. Even the production of a simple dipeptide such as Asp-Phe-me requires several protecting and deprotecting steps. Thus, although relatively high priced aspartame may be accepted by those whose health requires it such a high price places a limit on the commercial potential of this product. It would, therefore, be highly desirable to have an inexpensive and convenient method for producing aspartame.

Recent techniques have made possible the introduction of foreign genetic material into microorganisms which then produce the protein or proteins for which such foreign genetic material codes. The genetic code, which is based on sequence combinations of four possible nucleotide bases on the reading strand of a double-stranded DNA molecule, is now well known. Each sequence of three nucleotide bases is called a codon and for each specific amino acid, one or more codons exist. The four possible nucleotide bases of DNA are thymine, adenine, guanine and cytosine, which will hereinafter be referred to by their common abbreviations T, A, G, and C. The non-reading strand or complementary strand contains bases which are "complementary" to those in the reading strand. In the DNA molecule, C complements G, T complements A, G complements C, and A complements T.

It is known that the nucleotide base sequence GAC comprises a codon for aspartic acid (Asp). It is also known that the nucleotide base sequence TTT comprises a codon for phenylalanine (Phe). Inserting such codons in the DNA of a microorganism, preceded and followed by appropriate processing or termination codons, under appropriate control, and in the correct reading frame, would result in the microorganism producing the dipeptide Asp-Phe as part of its own protein producing processes.

Inserting a DNA segment coding for the dipeptide Asp-Phe as suggested above would, however, be commercially unsuited for the production of Asp-Phe. Because the natural digestive enzymes of an organism degrade or destroy the unnatural dipeptide, the likelihood of substantial product recovery is low. This is compounded by the fact that the sequence coding for Asp-Phe would represent only a minute fraction of the organism's DNA and significant amounts of Asp-Phe would not be produced.

An object of the invention is to provide an improved process for producing aspartame.

Another object is to provide a method for producing commercial quantities of aspartame using recombinant DNA.

Another object of the invention is to produce a microorganism from which substantial amounts of the dipeptide Asp-Phe may be derived.

The above objects are achieved by inserting into a cloning vehicle a synthesized DNA segment which codes for a protein segment of the formula $(Asp-Phe)_n$ where n is a large number. The resulting chimera is introduced into a living organism which in its changed form will produce a correspondingly large protein with the segment $(Asp-Phe)_n$. After benzylation of the free carboxyl groups, the protein is appropriately cut into dipeptide segments (Asp-Phe), methylated and debenzylated to form the peptide Asp-Phe-me which is useful as a sweetener.

So that the invention may be more fully understood, the invention will now be described in greater detail.

For a microorganism to produce a peptide having a long chain of the repeating sequence $(Asp-Phe)_n$ the organism must have a strand of DNA which has alternating codons which code for aspartic acid and phenylalanine. Furthermore, such a strand must be inserted in a DNA segment in an appropriate position relative to promoters and operators and in the correct reading frame so that the genetice code is transcribed to messenger RNA and translated to form the desired protein. The promotors and operators may be synthesized along with the sequence $(Asp-Phe)_n$ as part of the inserted strand, or may be part of the cloning vehicle in which the strand is inserted.

In the genetic code, the codons TTT and the codons TTC code of phenylalanine. The codons GAT and GAC code for aspartic acid. Thus, for example, a microorganism having a DNA segment in the reading strand $(GAS-TTT)_n$ properly located will produce a peptide with a long segment of the formula $(Asp-Phe)_n$.

DNA segments suitable for cloning are obtained from other organisms or must be synthesized. As there is no known natural source for a DNA strand which codes for the repeating protein sequence Asp-Phe, an appropriate DNA chain must be synthesized.

Single stranded DNA chains may be built-up in a stepwise method. A preferred method is a modified phosphotriester method described by K. Itakura, C. P. Bahl, N. Katagiri, J. Michniewicz, R. H. Wightman and S. A. Narang. Can. J. Chem. 51,3649 (1973). Such a synthesis may be used to produce a DNA chain with the exact sequence of nucleotide bases required.

A limitation on the modified triester method is that it is usually quite difficult to produce nucleotide base chains over about 15 or 20 bases. As it is desirable to insert a chain which codes for $(Asp-Phe)_n$ where n is large enough to confer stability on the protein, it is usually preferable to synthesize shorter nucleotide sequences and join them together.

A double stranded segment of DNA having the six nucleotide base sequence $\frac{(GAC-TTT)}{(CTG-AAA)}$ is polymerized end to end would result in a chain having the formula $\frac{(GAC-TTT)}{(CTG-AAA)_n}$. However, there is no suitable way to join a number of such chains together in invariably the correct order. For example, two such chains could join together to form the sequence $\frac{GAC-TTT-AAA-GTC}{CTG-AAA-TTT-CAG}$ instead of $\frac{GAC-TTT-GAC-TTT}{CTG-AAA-CTC-AAA}$. Accordingly, for reasons hereinafter more fully discussed, it is preferable to synthesize a pair of single stranded 12-base nucleotide chains. The first is the coding sequence GAC-TTT-GAC-TTT and the second is the sequence AAA-CTG-AAA-CTG. The 12-base coding nucleotide sequence GAC-TTT-GAC-TTT is not the only sequence which would alternately code for Asp and Phe because, as is well known in the art, there are two possible codons for both aspartic acid and for phenylalanine. However, the above 12-base coding chain is chosen for simplicity of synthesis.

The 12-base nucleotide coding chain may be formed by the stepwise addition of nucleotides as described by Narang, et al. However, it is simpler and thus preferable to form the six-base chains with the sequence GAC-TTT and join two such chains together. The dimerization of two six-base nucleotide chains is accomplished through the use of mesitylene sulfonyltetrazole as a coupling reagent, as described by J. Stawinski, T. Hozumi and S. A. Narang, Can. J. Chem., 54, 670 (1976). The six-base DNA chain is synthesized stepwise by the modified triester method.

The base sequence AAA-GTC-AAA-GTC is similarly formed. This sequence comprises a segment of the DNA strand complementary to the $(GAC-TTT)_n$ strand, but is offset in relation to the GAC-TTT-GAC-TTT segment. The reason for this will become apparent below.

DNA exists in nature primarily as double-stranded helical molecules. Base pair hydrogen bonding between adenine and thymine and between cytosine and guanine provide the binding force between a nucleotide chain and its complementary chain. Similarly, an artificially produced strand of nucleotide bases will in an appropriate solution attract its complementary chain and attach thereto by hydrogen bonding of complementary base pairs. Thus, in an appropriate solution a segment GAC-TTT will bind to the segment AAA-GTC.

Because of the repeating sequence in both synthesized strands of 12 nucleotides described above, there are three ways that they can pair.

```
    I.   5'                   GAC-TTT-GAC-TTT      3'
         3'      CTG-AAA-CTG-AAA                   5'

   II.   5'      GAC-TTT-GAC-TTT                   3'
         3'      CTG-AAA-CTG-AAA                   5'

  III.   5'      GAC-TTT-GAC-TTT                   3'
         3'                   CTG-AAA-CTG-AAA      5'
```

The manner of pairing is a random consequence of initial interaction of complementary nucleotides. The offset strands as in I and III may further bind with other 12-base single strands or polymerize with other offset double strands to form long hydrogen bonded nucleotide chains, i.e.:

```
              GAC-TTT (n)  GAC-TTT      GAC-TTT-GAC-TTT 'n)
(n) CTG-AAA-CTG-AAA        CTG-AAA (n)  CTG-AAA
```

The blunt end chain as in II above will not polymerize by hydrogen bonding. Although there are methods to join such chains, as for example with T4 ligase, there is no way to assure that the chains so produced will invariably form in the correct order.

One could assure that the 12-base segments would join in the offset manner by changing the codons of the second set of 6 nucleotides to the alternate codons for Asp-Phe.

The polymerized double chain formed by the hydrogen bonding of the complementary 12-base chains is not a complete DNA double strand as there is typically a break in the deoxyribose phosphate backbone every 12 nucleotides on each chain. The missing deoxyribose phosphate bonds are formed with DNA ligase to give a double stranded DNA segment having the formula of the type:

$$\begin{array}{c} (GAC\text{-}TTT)_n \ GAC\text{-}TTT \\ CTG\text{-}AAA \ (CTG\text{-}AAA)_n \end{array}$$

At each end of the double strand a 6 nucleotide base chain tail will be single stranded. This is converted to a double strand through the use of DNA polymerase in the presence of the appropriate deoxyribonucleotide triphosphates to achieve a blunt ended DNA chain.

$$\begin{array}{c} (GAC\text{-}TTT) \ GAC\text{-}TTT \\ CTG\text{-}AAA \ (CTG\text{-}AAA)_{n-2} \end{array} \xrightarrow[\text{DNA polymerase}]{GTP,ATP,CTP,TTP} \begin{array}{c} (GAC\text{-}TTT) \\ (CTG\text{-}AAA)_n \end{array} \cdot$$

The DNA segment $\dfrac{(GAC\text{-}TTT)}{(CAG\text{-}AAA)_n}$ has the correct base sequence to direct the production of the protein sequence $(Asp\text{-}Phe)_n$. However, in order for the protein to be produced it is necessary to insert the segment in a cloning vehicle and insert the cloning vehicle into a living organism.

Cloning vehicles are generally relatively simple DNA molecules which may be introduced into a microorganism and which function in the microorganism to direct the synthesis of protein. Appropriate cloning vehicles include plasmids and viruses such as lambda phages or SV 40 virus. Plasmids are non-nuclear DNA which in a microorganism replicate and direct the synthesis of protein. Viruses are a simple type of organism composed largely of DNA which lack independent ability to metabolize and reproduce. Viruses infect cells and will in most cases take over and eventually destroy a cell. Certain viruses such as lambda phages, however, may exist as lysogens in microorganisms and may be carried from one generation to another in the microorganisms.

While most double helixes of DNA exist as straight chains, many simple DNA strands such as viruses and plasmids are closed loops of DNA. Closed loops of DNA are most suitable as cloning vehicles. Becuase the DNA in the cloning vehicle must be cut in order that the artificial or foreign segment may be inserted, it is desirable that a small loop of DNA be used, so that the severed ends may remain in proximal relation to each other.

In order that the foreign DNA segment be inserted, the cloning vehicle must be cut. This is accomplished through the use of various restriction enzymes. Restriction enzymes recognize a particular nucleotide base sequence, usually a segment having a center of symmetry, and cut a double-stranded DNA chain in a predetermined manner. For example, the sequence 5' GAATTC 3' / 3' CTTAAG 5' is cut by EcoRl to form two severed ends as follows:

$$\begin{array}{cccc} 5' & G & AATTC & 3' \\ 3' & CTTAA & G & 5' \end{array}$$

The severed ends may rejoin by base pairing to each other or may join to chains having a single strand tail complementary to the single strand tail on the cut strand. Thus, for EcoRl the sequence -AATT is a recognition sequence for the EcoRl restriction site, the sequence -AATT being self complementary.

A restriction enzyme will cut the cloning vehicle wherever the recognized sequence appears. It is most desirable to use a restriction enzyme which cuts the cloning vehicle at a single site. If a circular cloning vehicle is cut at a single site, generally none of the genetic material of the cloning vehicle will be lost and hence will probably remain functional after insertion of the foreign segment and rejoining of the ends. A cloning vehicle may be useful which is cut by a restriction enzyme at more than one site providing that a remaining DNA fragment contains sufficient genetic material to be functional after insertion into a
microorganism.

The virus SV 40 is an example of a virus which
is cut by a restriction enzyme, i.e., EcoRl, at a single
site. Plasmids have also been developed by genetic
manipulation which are cut by a particular restriction
enzyme at a single site. A suitable plasmid for
insertion of an artificial DNA segment is pBGP120 which
was developed and described by B. Polisky, R. J. Bishop
and D. H. Gelfand, Proc. Natl. Acad. Sci. U.S.A., 73,
3900-3904 (1976). The plasmid pBGP120 was developed to
be split by the restriction enzyme EcoRl at a single
site so that after insertion of a foreign DNA segment
the ends could be rejoined to form a plasmid containing
all the original genetic material as well as all the
inserted foreign genetic material.

In order that protein synthesis be directed by
an inserted DNA segment, the inserted DNA must be
inserted so that it is under the direction of a promoter
and operator for mRNA transcription to occur. The
plasmid pBGP120 has its sole EcorI restriction site at
the distal end of most of the beta-galactosidase gene.
Foreign genetic material inserted at the EcoRI
restriction site is under the direction of the lac
promoter and operator. Transcription reads through the
beta-galactosidase gene into the inserted segment so
that inserted foreign genetic material will direct the
production of protein.

The inserted foreign segment must be in phase
for correct transcription and translation as the genetic
code is read in groups of 3. So that the foreign
segment will be in phase, the foreign segment must be
inserted 3n bases from the beginning of translation. If
inserted 3n+1 or 3n-1 bases from the beginning of
translation, the foreign segment will be out of phase.

Thus, a sequence XXX-GAC-TTT where XXX is a codon will be read XXX, GAC, TTT. However, if the inserted segment is out of phase, as for example, in XXX-Y-GAC-TTT where XXX is a codon and Y is an additional nucleotide base, the sequence will be read XXX, YGA, CTT, T -- etc. Out of phase insertions of foreign genetic material will result in production of "junk" protein and/or termination of translation.

The EcoRI site in the plasmid pBGP120 is in the middle of a pair of codons GAA-TTC which code for glutamic acid and phenylalanine and is split to form identical ends:

$$5' \quad G \qquad 3'$$
$$3' \quad CTTAA \quad 5'$$

A foreign segment may be inserted in an EcoRI cut pBGP120 plasmid if it has single stranded EcoRI recognition tail i.e. -AATT at eachs' end. To be in phase an additional $3n+1$ nucleotides must precede the coding sequence.

A preferred method for inserting DNA segments is through the use of adaptors for molecular cloning a described by C. P. Bahl, K. J. Marians, R. Wu, J. Stawinski, and S. A. Narang, Gene., 1, 81 (1976).

The polymeric DNA $\left\{ \begin{smallmatrix} 5' & GAC & TTT & 3' \\ 3' & CTG & AAA & 5" \end{smallmatrix} \right\}_n$ is adapted for insertion into the EcoRI site of pBGP120 by fusing a 12 nucleotide self complementary dodecanucleotide CATGAATTCATG using T4 ligase. The DNA is cut with EcoRI endonuclease, the resulting DNA has a $\begin{smallmatrix} 5' & AATTCATG & 3' \\ 3' & \quad GTAC & 5' \end{smallmatrix}$ sequence attached to each end of the polymeric DNA. This sequence puts the polymeric DNA in the desired frame with promoter and ribosome binding sites of beta-galactosidase.

In an appropriate solution the adapted foreign segment attaches by hydrogen bonding of the single stranded recognition sequences to each end of the cut cloning vehicle. DNA ligase completes the deoxyribose-phosphate backbone to reform a circular DNA cloning vehicle which will direct the synthesis of a protein having a segment with the repeating sequence $(Asp-Phe)_n$.

The cloning vehicle is inserted in a microorganism. For the plasmid pBGP120, a preferred microorganism is a strain of E. coli and particularly the well characterized strain of E. coli K12. Plasmids may be introduced into bacteria by methods such as those described by Cohen, et al., Proc. Natl. Acad Sci., 69 2110-2114 (1972). The microorganism in which the cloning vehicle is inserted produces along with its other proteins the desired protein which contains a long (Asp-Phe) segment. An E. coli organism containing a chimeric plasmid is cultured by methods for culturing E. coli well known in the art.

The desired protein is then harvested from the culture of cloned microorganism. If the desired protein is secreted by the microorganism, the protein may be drawn off in a solution such as a supernatant. If the protein is retained in the cells, the cells may be lysed and centrifuged to remove cell walls and other insoluble material. Small molecules are removed from the supernatant by appropriate methods such as dialysis or molecular sieve.

The $(Asp-Phe)_n$ protein segment is a long amino acid chain and is, of course, a repeating sequence. The repeating sequence is advantageously used in the protein purification. Several chemicals and enzymes are known which split protein chains at specific location. For example, CNBr splits protein on the carboxyl side of methionine. Trypsin splits proteins at the arginine or lysine moieties. Neither trypsin nor CNBr cuts the Asp-Phe or Phe-Asp bond. If the protein fraction is digested by either trypsin or CNBr, the proteins will be cut at each susceptible site. Hybrid protein attached to the $(Asp-Phe)_n$ segment is substantially eliminated and the other proteins fragmented, but the $(Asp-Phe)_n$ segment is uncut and is significantly larger than any of the resulting peptide fragments. The long chain $(Asp-Phe)_n$ is removed from the short peptide fragments by methods such as ultra centrifugation or filtration through an appropriate sized molecular sieve.

Because the carboxyl group of the phenylalanine is to be esterified and because the aspartic acid has a free carboxyl group, the carboxyl group of the aspartic acid is protected with a benzyl group or a substituted benzyl group which is to be removed later by hydrogenation. The protected protein is digested with chymotrypsin which cuts the $(Asp(B_2)-Phe)_n$ chain into the protected dipeptide $(Asp)(B_2)-Phe)$. The protected dipeptide is methylated with an excess of methanol to produce $Asp(B_2)-Phe-Me$. Hydrogenolysis removes the benzyl group to produce the desired methylated dipeptide Asp-Phe-Me.

The described method of producing aspartame allows the artifical sweetener to be cheaply produced in large quantities. While the production of an altered microorganism as described above is a long and tedious procedure, once a microorganism which produces $(Asp-Phe)_n$ is developed, so long as the strain is kept alive, the microorganism forming procedure need not be repeated. The microorganism can be grown in large batches analogous to the production of yeast. The living cells do not need purified amino acids as is required in the stepwise production of peptides but only requires simple growth media providing a source of

- 14 -

carbon, nitrogen, phosphorus and simple salts. The protein purification steps are relatively simple and are adaptable to industrial techniques known to those skilled in the art.

Although the invention has been described with regard to certain preferred embodiments, it is to be understood that the invention includes modifications obvious to one skilled in the art. For example, while the invention is described in terms of preferred cloning vehicles and in terms of preferred host organisms, the invention includes any suitable cloning vehicle and any suitable host.

CLAIMS:

1. A method for producing aspartame, comprising, synthesizing double stranded DNA in which a coding strand has alternating codons for Asp and Phe, said codons being of a number sufficient to produce a polypeptide which is stable in a predetermined host microorganism, inserting said DNA strand into a cloning vehicle so that resulting chimera directs the synthesis of said protein, introducing said chimera into said predetermined host microorganism and cultivating said microorganism to produce said stable polypeptide, harvesting said stable polypeptide from said host microorganism esterifying the free carboxyl group of aspartic acid by benzylation, fragmenting said polypeptide to produce benzyl-Asp-Phe dipeptides, methylating the carboxyl group of the Phe moiety, and debenzylating the aspartic acid carboxyl group by hydrogenolysis.

2. A method according to Claim 1 wherein the carboxyl group of the Phe moiety is methylated by protecting the carboxyl group of Asp, breaking the Phe-Asp bond, methylating the carboxyl group of the Phe moiety, and deprotecting the carboxyl group of the Asp moiety.

3. A method according to Claim 2 wherein said carboxyl group is protected by esterification.

4. A method according to Claim 2 wherein said carboxyl group is protected with a benzyl group or substituted derivatives thereof.

5. A method according to Claim 2 wherein said deprotection is accomplished by hydrogenolysis.

6. A method according to Claim 1 wherein said polypeptide is fragmented by digestion in a medium which

16.  0036258

breaks specific peptide links not including Asp-Phe or
Phe-Asp bonds.

7. A method according to claim 6 wherein said medium
contains CNBr.

8. A method according to claim 6 wherein said
medium includes an enzyme which breaks said specific
peptide links.

9. A method according to claim 8 wherein said
medium contains trypsin.

10. A method according to claim 1 wherein said host is
E.coli.

11. A method according to claim 1 wherein said
host is B.subtilis.

12. A method according to claim 1 wherein said
host is E.coli. K12.

13. A method for producing aspartame, comprising,
cultivating a microorganism which produces a protein
with a segment having a sequence $(Asp-Phe)_n$, harvesting
said protein segment, esterifying the free carboxyl group
of aspartic acid by benzylation, fragmenting said polypeptide
to produce benzyl-Asp-Phe dipeptides, methylating the the carboxyl group of the Phe moiety, and debenzylating the aspartic acid carboxyl group by hydrogenolysis.


14. A microorganism which produces a protein with a segment having a sequence $(Asp-Phe)_n$.


15. A microorganism according to claim 14 wherein said microorganism is a strain of E.coli.


16. A microorganism according to claim 14 wherein said microorganism is a strain of E.coli Kl2.


17. A microorganism according to claim 14 wherein said E.coli contains a plasmid which directs the synthesis of said protein.


18. A microorganism according to claim 14 wherein said microorganism is a strain of B.subtilis.


19. A microorganism according to claim 14 wherein said B.subtilis contains a plasmid which directs the synthesis of said protein.